# EUROPEAN PATENT SPECIFICATION

(11) **EP 3 583 934 B1**
(45) Date of publication and mention of the grant of the patent: **25.08.2021**
(21) Application number: 18794421.0
(22) Date of filing: 29.01.2018
(51) Int. Cl.: A61K 38/10, A61P 31/00, A61P 31/22, A61K 9/06, A61K 47/32

(54) **BIOCIDAL AGENT**
BIOZID
AGENT À EFFET BIOCIDE

(30) Priority: 05.05.2017 RU 2017116079
(43) Date of publication of application: 25.12.2019
(73) Proprietor: "Verta Research-Production Company" Ltd, St.Petersburg 197110 (RU)
(72) Inventor: AFONINA, Irina Vasil'evna, St.Petersburg 198013 (RU); KOLOBOV, Alexandr Alexandrovich, St.Petersburg 197706 (RU); KOLODKIN, Nikolay Ivanovich, St.Petersburg 196084 (RU); SMIRNOVA, Mariya Pavlovna, St.Petersburg 197183 (RU); STEFANENKO, Ludmila Ivanovna, St.Petersburg 194362 (RU)
(74) Representative: Jeck, Anton
(86) International application number: PCT/RU2018/000039
(87) International publication number: WO 2018/203772

(56) References cited:
- EP-A1- 3 578 566
- WO-A1-2008/007997
- WO-A2-2004/050132
- RU-C1- 2 172 322
- SMIRNOVA MP. et al.: "Structure-function relationship between analogues of the antibacterial peptide indolicidin. I. Synthesis and biological activity of analogues with increased amphipathicity and elevated net positive charge of the molecule", Bioorg Khim., vol. 30, no. 5 September 2004 (2004-09), pages 409-465, XP055561923, [retrieved on 2018-04-25]
- SITARAM N. et al.: "Indolicidin, a 13-residue basic antimicrobial peptide rich in tryptophan and proline, interacts with Ca(2+)-calmodulin", Biochem Biophys Res Commun., vol. 309, no. 4, 3 October 2003 (2003-10-03), pages 879-884, XP004455823,

## Description

### Field of the Invention

The invention relates to the field of medicine, in particular, to novel pharmaceutical compositions having biocidal properties, more particularly, antimicrobial and antiviral properties.

### Prior Art

According to the WHO, about 80% of the world's population are infected with various types of herpes simplex virus (HPV). One of the most severe and socially significant HPV-caused diseases is herpetic lesions of the genital area, predominantly caused by type 2 HPV. According to the 2015 data, more than 410 million people 15 to 50 years of age are infected with HPV-2. Herpetic lesions not only reduce the quality of life of those infected, but also significantly disrupt the barrier function of the epithelium, which combined with a number of other pathogenic mechanisms increases the risk of HIV infection (Freeman E.E. et al., AIDS. 2006, Jan 2;20(I):73-83; Freedman E., Mindel A., J. HIV Ther., 2004, Feb; 9(I):4-8).

Natural killers (NK cells) were found to play an important role in suppressing the HPV-activity, and their malfunction or taxis impairment in the affected area causes an increased HPV activity and higher incidence of relapses (Orange J. S., Microbes Infect., 2002, 4:1545-1558; Murugin V. et al., Clin. Vaccine Immunol., 2011, Sep; 18(9): 1410-1415). Besides NK cells, T-lymphocytes also play an important role in the fight against HPV, in particular cytotoxic CD8+T-cells. *In vivo* models demonstrated that the absence of CD8+T-cells significantly exacerbates the course of infection (Chayavichitsilp P. et al., Pediatr. Rev., 2009, 30:119-130). Patients with malfunctioning CD8+T-cells present with significantly more severe herpetic lesions and considerably higher incidence of relapses (Whitley R. J., Roizman B., Lancet, 2001, 357:1513-1518).

No drugs available on today's market can completely eliminate HPV from a human body. The virus survives in the regional nerve ganglion; and during relapses, which can occur six or more times a year, it moves to the epithelium, where it enters the replication stage, which is usually manifested as vesicular rash and ulcerations (O.I. Letyaeva et al RMJ, 2015, No28, pp 1701-1704). That is often followed by concomitant herpetic and bacterial infections.

Finding novel approaches to the treatment of said condition, therefore, remains an acute problem due to the diverse clinical manifestations and developed resistance of the virus to existing pharmaceutical preparations.

The most common drugs used for the treatment of HPV are synthetic acyclic nucleosides that disrupt HPV virus replication, such as Acyclovir, (Elion, G.B. et.al., PNAS, 1977, 74:5716-5720). The main disadvantage of this treatment is its low efficacy at the inactive stage of the disease. (T.B. Semyonova, RMJ, 2002. JVS 20. pp. 924-930).

Alloferons, and in particular Allokin-alpha (http://go.mail.ru/), which is the closest analog to the claimed invention, are known in the art as the peptides used for the treatment of HPV. Allokin-alpha is a synthetic oligopeptide having the structure of the bioactive peptide of insects alloferon 1. It is currently the only preparation from the alloferon class registered in Russia and used for this indication (RU 2172322, 2001; Registration Certificate No. 002829/01 on 09.22.2003). Said peptide is a linear oligopeptide with a molecular weight of 1265.3 Da, consisting of 13 amino acid residues with the following sequence:
NH₂-His-Gly-Val-Ser-Gly-His-Gly-Gln-His-Gly-Val-His-Gly-COOH

The therapeutic effect of the preparation was studied, iner alia, *in vivo* models. Experimental studies of the antiviral effect thereof on the most dangerous type of herpesvirus, human herpes simplex virus type 2 (HPV-2), were carried out. The model of genital herpes in male rabbits caused by an HPV-2 surface infection demonstrated that local application of Alloferon significantly shortened the length of the primary genital herpes outbreak and reduced the viral load on the body. Similar data was obtained in the experiments with HPV-2 in guinea pigs. The experiments demonstrated a statistically significant reduction in the disease duration and in the discharge of the virus from the lesions.

The disadvantages of said preparation are the need for parenteral administration and insufficient efficacy, especially against concomitant infections, due to the lack of direct impact on viruses and microorganisms.

Also WO2008007997 describes compositions comprising alloferon 1 for topical antiviral application.

The problem to be solved by the inventors was to create a more effective drug for the treatment of HPV, including the treatment of HPV complicated by a bacterial infection.

Said problem was solved by creating a product for local application comprising a mixture of peptides: Alloferon 1 **H₂N-His-Gly-Val-Ser-Gly-His-Gly-Gln-His-Gly-Val-His-Gly-COOH (1)** and the indolicidin analog **H₂N-(CH₂)10-CO-Ile-Leu-Pro-D-Phe-Lys-D-Phe-Pro-D-Phe-D-Phe-Pro-D-Phe-Arg-Arg-NH₂ (2)** in a 1:2 to 2:1 mass ratio, as well as excipients. The optimum results are achieved when the mass ratio between the aforementioned peptides is 1:1.

The composition is prepared by mixing the ingredients together.
The special aspect of the proposed composition is the discovered effective combination of biocidal peptide (2), having a broad spectrum of action, and peptide (1) exhibiting an immunomodulatory effect and stimulating both NK-cells and cytotoxic T-lymphocytes.

The pharmaceutical preparation was prepared using standard techniques for the preparation of dosage forms of pharmaceuticals for local application, using a pharmaceutically acceptable salt of an organic or inorganic peptide that doesn't cause any adverse local and systemic side effects as the active ingredient. Appropriate additives providing structural viscosity and having pseudoplastic, plastic, and thixotropic properties, which optimize the bioavailability of the active ingredients, were used as excipients.

The peptides for this pharmaceutical preparation were prepared using a technique for sequential peptide chain elongation on a polymer carrier, followed by identification and purity determination of the obtained product by high-performance liquid chromatography (HPLC) and mass-spectrometry.

Based on the aforementioned peptides, a gel preparation for local application with biocidal effect was prepared. Said gel has the following composition (wt. %): peptides-0.02-0.2 and auxiliary additives (excipients) - 99.98-99.8. As excipients, the gel may include Carbopol, allantoin, phenoxyethanol, ethylhexylglycerin, sodium hydroxide, sodium hyaluronate, lavender or castor oil, alcohol, distilled glycerin, sodium carmellose, citric acid, alpha-tocopherol acetate, benzoic acid, flavors, etc. The end product is a transparent homogeneous gel for external (local) application free of foreign particles.

As the conducted experiments demonstrated, the claimed preparation is most promising for the external treatment of skin and mucosa infected with bacteria, fungus, herpes virus, and papillomavirus, and in particular, genital infectious and inflammatory diseases, and also for the prevention of obstetric and gynecological infectious inflammatory complications, and similar conditions.

The practical applicability of the invention is illustrated by the following examples:

### Example 1: Synthesis peptide of NH₂-His-Gly-Val-Ser-Gly-His-Gly-Gln-His-Gly-Val-His-Gly-COOH (1)

The tert-butyloxycarbonyl group was used as a temporary protective group. The permanent protective groups were dinitrophenyl on histidine and benzyl ether on serine. The synthesis was conducted on a PAM-polymer with 0.8 mmol/g capacity. The peptide chain was further elongated from the C-terminus following the procedure in Table 1.

**Table 1. Procedure of adding one amino acid residue**

| No p/p | Operation | Reagents | Repetitions | Time, min | Reagent volume, ml |
|---|---|---|---|---|---|
| 1 | Washing | Dichloromethane | 1 | 2 | 10 |
| 2 | Unblocking | Trifluoroacetic acid | 1 | 2 | 5 |
| 3 | Unblocking | Trifluoroacetic acid | 1 | 2 | 5 |
| 4 | Washing | Dimethylformamide | 3 | 1 | 10 |
| 5 | Neutralization | Diisopropylethylamine (5 mmol in dimethylformamide) | 1 | 1 | 5 |
| 6 | Condensation | 1.0 mmol oxybenzotriazole ester Bocderivative of amino acid containing 1.5 mmol diisopropylethylamine | 1 | 30 | 5 |
| 7 | Washing | Dimethylformamide | 3 | 1 | 10 |
| 8 | Ninhydrin test/bromophenol blue-Pro/ | | | | |

If the ninhydrin test was positive, the cycle was repeated starting with p. 3.

Upon completion of the synthesis, the peptidyl-polymer was treated with 5 ml of 50 % trifluoroacetic acid in methylene chloride, washed with 5 ml of methylene chloride, and removed from the vessel. The product was washed on a filter thrice with 5 ml of isopropyl alcohol portions and thrice with 5 ml of absolute ether portions. The obtained peptidyl-polymer was dried in a vacuum-exicator over phosphorus pentoxide to constant weight, treated with 5% 2-merkaptoethanol in dimethylformamide, and finally unblocked while being simultaneously cleaved from the polymer with liquid hydrogen fluoride according to the procedure in Table 2.

**Table 2. Procedure for unblocking peptidyl-polymer and cleaving the peptide from the polymeric matrix**

| N p/p | Operation | Reagents | Repetition rate | Time, min | Temperature, °C | Reagent volume, ml |
|---|---|---|---|---|---|---|
| 1 | Total unblocking and cleavage of the peptide from polymeric matrix | Anhydrous hydrogen fluoride/m-cresol (10:1 by volume) | 1 | 60 | 0 | 5 |
| 2 | Evaporation | | | | | |
| 3 | Washing, filtration | Absolute ether | 3 | 5 | | 25 |
| 4 | Dissolving | Trifluoroacetic acid | 3 | - | - | 1 |
| 5 | Precipitation | Diethyl ether | | | | 100 |
| 6 | Filtration | | | | | |
| 7 | Washing | Diethyl ether | 3 | | | 10 |

The obtained crude product was purified by reverse-phase chromatography on a C18 Nova Pack column, 19x300 mm, 5-50% acetonitrile gradient in 0.1% trifluoroacetic acid, collecting the fractions containing the final product. According to the optical density data, the content of the main substance, peptide NH₂-His-Gly-Val-Ser-Gly-His-Gly-Gln-His-Gly-Val-His-Gly-COOH was at least 99%. The amino acid composition of the peptide and its molecular weight corresponded to the theoretical values.

### Example 2: Synthesis of biocidal peptide H₂N-(CH₂)₁₀-CO-Ile-Leu-Pro-D-Phe-Lys-D-Phe-Pro-D-Phe-D-Phe-Pro-D-Phe-Arg-Arg-NH₂ (2)

The synthesis, unblocking, and purification of peptide (2) were conducted using the same method as that for the peptide in Example 1. To protect the N^{ω}-function of arginine, the nitro group from the ω-amino group of lysine, the chorocarbobenzoxy group, was used. To synthesize the peptide, 0.2 g of N-tert-butyloxycarbonyl-N_{ω}-nitroarginine methylbenzhydryl amino polymer and 10 ml of dimethylformamide were added to a reaction vessel. The content of the arginine derivative was 1.0 mmol/g of the polymer.

Upon completion of the polypeptide chain assembly, the final unblocking of the protected peptidyl-polymer with simultaneous cleavage of the peptide from the polymer with liquid hydrogen fluoride were conducted. Purification was conducted with an acetonitrile gradient of 10-90% in 0.1% trifluoroacetic acid. According to the optical density data, the content of the main substance, peptide NH₂-(CH₂)₁₀-CO-Ile-Leu-Pro-D-Phe-Lys-D-Phe-Pro-D-Phe-D-Phe-Pro-D-Phe-Arg-Arg-NH₂ was at least 99%. The amino acid composition of the peptide and its molecular weight corresponded to the theoretical values.

### Example 3. Preparation of gel for the prevention and treatment of skin and mucosa diseases.

The gel is prepared in a chemical reactor with a stirrer. Gel components are loaded into the mixer in the following formulation (wt.%): Carbopol (1.5-2.0%), distilled water, and NaOH solution (1.5-2.0%); mixed, allowed to stand for 30 min, titrated with citric acid solution to pH 6, and, while stirring, combined with portions of the aqueous peptide solution containing methylparaben in the amount of 0.01-0.2 wt.% and propylparaben in the amount of 0.002-0.01 wt.% as preservatives. 1.5-2.5 wt.% glycerin is added to the obtained mixture. The system is stirred for 10 min. and the pH is measured. The obtained homogeneous gel is left to mature for 24 hrs. The gel is then stirred again, and the homogeneous mass is packed into containers (tubes).

The end product is a transparent homogeneous gel for external (local) application free of foreign particles. The gel has the following composition (wt.%): peptide: 0.02-0.2 and auxiliary additives 99.999-99.9. The characteristics of the obtained gel preparations comprising peptides are shown in Table 3.

**Table 3. Composition of gel preparations comprising peptides**

| Sample | Active ingredient | | Excipients, wt.% (water-rest) | | | |
|---|---|---|---|---|---|---|
| | Peptide | Concentration Wt.% | Carbopol | Methylparaben | Propylparaben | Glycerin |
| I | 1 | 0.01 | 1.5 | 0.2 | 0.01 | 2.5 |
| | 2 | - | | | | |
| II | 1 | - | 2.0 | 0.01 | 0.002 | 1.8 |
| | 2 | 0.01 | | | | |
| III | 1 | 0.01 | 2.0 | 0.01 | 0.002 | 1.9 |
| | 2 | 0.01 | | | | |
| IV | 1 | 0.1 | 1.7 | 0.1 | 0.01 | 1.7 |
| | 2 | 0.1 | | | | |
| V | 1 | 0.05 | 1.9 | 0.1 | 0.018 | 2.0 |
| | 2 | 0.1 | | | | |
| VI | 1 | 0.1 | 1.7 | 0.1 | 0.019 | 1.7 |
| | 2 | 0.05 | | | | |

### Example 4. Biocidal effect of gels on bacteria

The antimicrobial effect was determined by the radial diffusion method in agarose gels (AG). A lower AG layer (10 mM of sodium phosphate and 0.3 mg of soya broth hydrolysate powder per ml of 1% agarose) was prepared and 4x10⁶ CFU in the mean logarithmic growth phase were dispersed thereupon.

Series of gel samples with known peptide concentrations were prepared by suspending peptide-containing gels in 0.01% acetic acid comprising 0.1% human serum albumin. 10-µl of the experimental sample were applied to the lower AG layer. Three hours after the peptide application, the top AG layer was applied (10 ml of soya agar hydrolysate, 60 g/l). Following one night of incubation, the lightened zones were measured with 0.1 mm accuracy. The peptide activity was expressed in relative units of 1 mm=10 U. MIC was determined as segment lengths on the X-axis of the regression line of zone diameters obtained by serial dilutions of peptide-containing gels. The biocidal effect test results of the obtained gels calculated to the peptide 2 concentration are given in Table 3. Gel I exhibited no biocidal effect on the used microorganism strains.

**Table 3**

| Biocidal effect of gels on bacteria | | | | | | |
|---|---|---|---|---|---|---|
| MIC, µ/ml | Microorganism | Gel | | | | |
| | | II | III | IV | V | VI |
| | *E.coli ML-35p* | 1.8 ± 0.4 | 0.9 ± 0.2 | 0.9 ± 0.1 | 0.8 ± 0.3 | 1.2 ± 0.4 |
| | *Pseudomonas aeruginosa ATCC* 27853 | 3.5 ± 0.2 | 3.2 ± 0.2 | 2.0 ± 0.3 | 2.3 ± 0.3 | 3.2 ± 0.5 |
| | *St. aureus MR ATCC33591* | 8.0 ± 0.5 | 6.0 ± 0.3 | 6.5 ± 0.5 | 6.2 ± 0.6 | 7.3 ± 0.6 |
| | *Enterococcus faecium L3* | 10.1 ± 0.3 | 4.0 ± 0.4 | 3.0 ± 0.6 | 3.3 ± 0.5 | 5.1 ± 0.4 |
| | *Bacillus subtilis* | 3.0 ± 0.2 | 0.9 ± 0.1 | 0.7 ± 0.1 | 1.2 ± 0.3 | 2.8 ± 0.2 |
| | *Listeria monocytogenes EGD* | 3.5 ± 0.2 | 1.8 ± 0.2 | 1.3 ± 0.2 | 2.0 ± 0.4 | 2.5 ± 0.3 |

### Example 5. Antiviral effect of gels on human herpes simplex virus, type 2, in experiments in vivo.

Eight groups of animals, containing 15 subjects each, were formed to conduct the experiment.

Groups 1-6: infected with type 2 herpes simplex virus and administered gels I-VI (experimental groups 1-6).

Group 7: infected with type 2 herpes simplex virus and administered Acyclovir (preparation control).

Group 8: infected with type 2 herpes simplex virus and not administered any pharmaceutical preparations (virus control).

Type 2 herpes simplex virus, strain G (ATCC VR-734, USA), was used in the experiment. The virus was cultivated in the T98G cell culture (human brain glioblastoma), then adapted to mice by two consecutive intracerebral passages. To prepare the final infective material, the animals were intracerebrally infected with brain tissue homogenate from the second passage; on the 4^{th} day post infection, the animals were euthanized, their brain was extracted, homogenized, used to infect Vero cells, and incubated for 48 hours at 37°C under 5% CO₂ using the culture fluid, in which the infectious titer of the virus was previously determined, as the infective material. Zovirax (Acyclovir as 5% ointment, Glaxo Welcome) was used as a comparator drug.

The murine vaginal epithelium was injured with a scarifier for vaginal smears, after which the animals were infected with 30 µl of the virus at a 10⁶ TCID₅₀ dose. The examined gels were administered to mice in groups 1 and 2 per the following regimen: 0.05 ml once a day 1, 2, 3, 4, and 5 days post infection.

The animals were followed for 18 days; their mortality was recorded daily. Cessation of mortality was defined by the absence of mice with signs of herpetic vaginitis (vaginal discharge) and complications thereof (posterior limb paresis). Based on the mortality data for each group, the mortality rate (Mr, ratio of the fallen animals at the time of control to the total number of infected animals in the group), protective index (IP, ratio of the difference in the percentage of mortality in the control and experimental groups to the percentage of mortality in the control group), and average life span of the animals (ALS) per each day of the experiment were calculated.

The protective effect of the peptide-containing gels was assessed by the reduction in the specific mortality and increase in the life span of the animals compared to the control group that wasn't treated. The protective effect data is shown in Table 8.

**Table 8. Protective effect of gels in lethal herpes infection caused in mice by vaginal infection with type 2 herpes simplex virus (n=15)**

| Gel | Number of animals in group | Number of dead animals | Number of survived animals | ALS, 24 hrs. | Mortality, % | Protective index, % | Increase in ALS |
|---|---|---|---|---|---|---|---|
| I | 15 | 10 | 5 | 11.9 | 66.6 | 9.1 | 0.6 |
| II | 15 | 5 | 10 | 13.7 | 33.3 | 54.5 | 2.4 |
| III | 15 | 3 | 12 | 14.3 | 20.0 | 72.7 | 3.0 |
| IV | 15 | 2 | 13 | 14.6 | 13.3 | 81.8 | 3.3 |
| V | 15 | 2 | 13 | 14.0 | 13.3 | 81.8 | 3.0 |
| VI | 15 | 3 | 12 | 13.9 | 20.0 | 72.7 | 2.9 |
| Acyclovir | 15 | 4 | 11 | 13.9 | 26.7 | 63.6 | 2.6 |
| Virus control | 15 | 11 | 4 | 11.3 | 73.3 | - | 0.0 |

The conducted studies demonstrated that the claimed agent exhibited a combined antibacterial and antiviral effect and is a promising treatment of vaginal herpes infections including those complicated with bacterial infections.

## Claims

1. An agent for local application exhibiting a biocidal effect, comprising a peptide **H₂N-His-Gly-Val-Ser-Gly-His-Gly-Gin-His-Gly-Val-His-Gly-COOH** and excipients, wherein said agent additionally comprises a peptide **H₂N- (CH₂)₁₀-CO-Ile-Leu-Pro-D-Phe-Lys-D-Phe-Pro-D-Phe-D-Phe-Pro-D-Phe -Arg-Arg-NH₂,** wherein the ratio between said peptides is from 1:2 to 2:1 (wt. %)

2. The agent of claim 1, wherein said agent comprises a mixture of peptides in a 1:1 mass ratio.

3. The agent of claim 1, wherein the peptide content in the mixture is 0.2 to 0.002 wt.%.

## Patentansprüche

1. Mittel mit biozider Wirkung zur lokalen Anwendung, aufweisend ein Peptid-**H₂N-His-Gly-Val-Ser-Gly-His-Gly-Gln-His-Gly-Val-His-Gly-COOH** und Hilfsstoffe, wobei das Mittel zusätzlich ein Peptid **H₂N-(CH₂)₁₀-CO-Ile-Leu-Pro-D-Phe-Lys-D-Phe-Pro-D-Phe-D-Phe-Pro-D-Phe-Arg-Arg-NH₂** aufweist und wobei das Verhältnis zwischen den Peptiden 1: 2 bis 2:1 (Gew .-%) beträgt

2. Mittel nach Anspruch 1,
wobei das Mittel eine Mischung von Peptiden in einem Massenverhältnis von 1:1 umfasst.

3. Mittel nach Anspruch 1, wobei der Peptidgehalt in der Mischung 0,2 bis 0,002 Gew.-% beträgt.

## Revendications

1. Moyen avec effet biocide pour l'application topique présentant un peptide- **H₂N-His-Gly-Val-Ser-Gly-His-Gly-Gln-His-Gly-Val-His-Gly-COOH** et adjuvants où le moyen présente en outre un peptide **H₂N-(CH₂)₁₀-CO-Ile-Leu-Pro-D-Phe-Lys-D-Phe-Pro-D-Phe-D-Phe-Pro-D-Phe-Arg-Arg-NH₂** et où le rapport entre les peptides s'élève à 1: 2 jusqu'à 2:1 (poids-%);

2. Moyen selon la revendication 1 où le moyen comporte un mélange de peptides dans un rapport de masse 1:1;

3. Moyen selon la revendication 1 où le contenu du peptide s'élève dans le mélange à 0,2 jusqu'à 0,002 poids-%.
